# EUROPEAN PATENT APPLICATION

(11) **EP 4 644 519 A1**
(43) Date of publication of application: **05.11.2025**
(21) Application number: 23909923.7
(22) Date of filing: 30.11.2023
(51) Int. Cl.: C12M 1/00, C12N 5/00, B04B 5/04

(54) **DOUBLE-PUMP HARVESTING METHOD AND APPARATUS**

(30) Priority: 28.12.2022 CN 202211701908
(71) Applicant: Shenzhen Cellbri Bio-Innovation Technology Co., Ltd., Shenzhen, Guangdong 518107 (CN)
(72) Inventor: GUO, Xiaoliang, Shenzhen, Guangdong 518107 (CN); SHANG, Yuanfang, Shenzhen, Guangdong 518107 (CN); ZHANG, Jinxin, Shenzhen, Guangdong 518107 (CN); LIU, Fujing, Shenzhen, Guangdong 518107 (CN)
(74) Representative: Herrero & Asociados, S.L.
(86) International application number: PCT/CN2023/135468
(87) International publication number: WO 2024/139993

(57) **Abstract**

The present disclosure provides a dual pump harvesting process including a concentrating process and a formulating process. Two pumps are used to perform the concentrating process. One end of a first pump of the two pumps is connected to an initial sample container and an other end of the first pump is connected to a centrifugal container; one end of a second pump of the two pumps is connected to the centrifugal container and an other end of the second pump is connected to an intermediate container and a waste liquid container. A flow rate difference between the two pumps during concentration is set to be a flow rate threshold, and a flow rate of the second pump is higher than that of the first pump to perform a continuous flow concentration. The present disclosure also provides a dual pump harvesting apparatus.

## Description

### CROSS-REFERENCE TO RELATED APPLICATIONS

This application claims priority to Chinese patent application No. 202211701908.6 filed on December 28, 2022, entitled "A dual pump harvesting method and apparatus", the entire contents of which are incorporated herein by reference.

### FIELD

The disclosure relates to the technical field of bioengineering, and more particularly, to a dual pump harvesting method and apparatus.

### BACKGROUND

With the continuous improvement of medical technology, the contemporary medical field gradually moves from molecular therapy to cell therapy. Cell therapy refers to the transplantation or infusion of normal or bioengineered human autologous or allogeneic cells into the patient's body such that the newly-injected cells can replace the original damaged cells, or exert a stronger immune-killing function, to restore the function of diseased cells or enhance the ability of immune cells to fight specific diseases.

The current process for producing cell-based drugs includes cell culturing, concentration, and washing. Cells that have been concentrated and washed need to be formulated and dispensed when being used for cryopreservation, re-infusion, assaying, etc. When the number and the volume of the dispensing bag is large, manual calculation and operation become cumbersome, complicated, inefficient, and costly.

### SUMMARY

The present disclosure provides a dual pump harvesting method and apparatus.

In a first aspect, the present disclosure provides a dual pump harvesting method that includes a concentrating process and a formulating process, and the concentrating process includes:

using two pumps to perform the concentrating process; wherein one end of a first pump of the two pumps is connected to an initial sample container and an other end of the first pump is connected to a centrifugal container; one end of a second pump of the two pumps is connected to the centrifugal container and an other end of the second pump is connected to an intermediate container and a waste liquid container; setting a flow rate difference between the first pump and the second pump to be a flow rate threshold, and setting a flow rate of the second pump to be higher than that of the first pump to perform a continuous flow concentration.

In an embodiment, the flow rate threshold is 100-200 mL/min.

In an embodiment, the flow rate of the first pump is 100-200 mL/min, and/or the flow rate of the second pump is 200-300 mL/min.

In an embodiment, the first pump extracts an initial sample from the initial sample container into the centrifugal container for centrifugation, and the second pump pumps a supernatant in the centrifugal container into the waste liquid container, thereby forming a circulating continuous flow concentration until a remaining volume of a cell fluid in the centrifugal container is lower than a concentration threshold. In an embodiment, the concentration threshold is 70-150 mL.

In an embodiment, during a process of the circulating continuous flow concentration, a concentration volume is preset, and a number of concentration cycles is determined based on the concentration volume; for example, if the preset concentration volume is 1000 mL, the number of concentration cycles = a volume of initial sample/1000 mL, rounded down.

In an embodiment, the concentrating process further includes determining a concentration way based on a volume of the initial sample; when the volume of the initial sample is greater than a preset multiple of a volume of the initial sample container, the circulating continuous flow concentration is used, otherwise a standard continuous flow concentration is used.

In an embodiment, the preset multiple is 12.

In an embodiment, during the concentrating process, the initial sample in the initial sample container is transferred into the centrifugal container; when an amount of the initial sample remaining in the initial sample container is reduced to a preset percentage, a bubble sensor is turned on.

In an embodiment, the preset percentage is 30%.

In an embodiment, the formulating process includes adding a first preset volume of a resuspension into the centrifugal container to resuspend cells and then determining the formulating process according to a preset total formulation volume; when the preset total formulation volume is less than 250 mL, a single cycle formulating process is used; when the preset total formulation volume is greater than 250 mL, a circulating formulating process is used.

In an embodiment, the circulating formulating process includes adding a first preset volume of formula solution to the intermediate container; transferring resuspended cells in the centrifugal container to the intermediate container; cleaning the centrifugal container and transferring a used cleaning solution to the intermediate container; adding a second preset volume of the formula solution to the intermediate container such that a total volume of a cell fluid in the intermediate container is equal to a sum of the preset total formulation volume and a compensation volume; and
dispensing the cell fluid in the intermediate container according to a preset dispensing quantity and a preset dispensing volume.

In a second aspect, the present disclosure provides a dual pump harvesting apparatus including a first pump and a second pump. One end of the first pump is connected to an initial sample container and an other end of the first pump is connected to a centrifugal container; one end of the second pump is connected to the centrifugal container and an other end of the second pump is connected to an intermediate container and a waste liquid container. A flow rate difference between the second pump and the first pump during concentration is set to be a flow rate threshold, and a flow rate of the second pump is higher than the first pump to perform a continuous flow concentration.

In a third aspect, the present disclosure provides a cell formulating system that includes the dual pump harvesting apparatus.

The present disclosure has the following beneficial effects:
Through a specific dual-pump process, the present disclosure realizes the circulating continuous flow concentration during the concentrating and the formulating processes under a relatively-high flow rate. Combined with the subsequent circulating formulating process, the efficiency and accuracy of the formulating process can be significantly improved. Each cycle of the formulating process can prepare a maximum of 9 products, which is higher than the existing preparation efficiency. The dual-pump harvesting method provided by the present disclosure can significantly improve the production efficiency of cell drugs and is important.

### BRIEF DESCRIPTION OF THE DRAWINGS

To more clearly illustrate the technical solutions in the present disclosure or prior art, a brief description of the accompanying drawing to be used in the description of the embodiments or prior art will be given below. The drawing described below is obviously just an embodiment of the present disclosure. For ordinary technicians in this field, other drawings can be obtained based on the drawing without doing creative work.

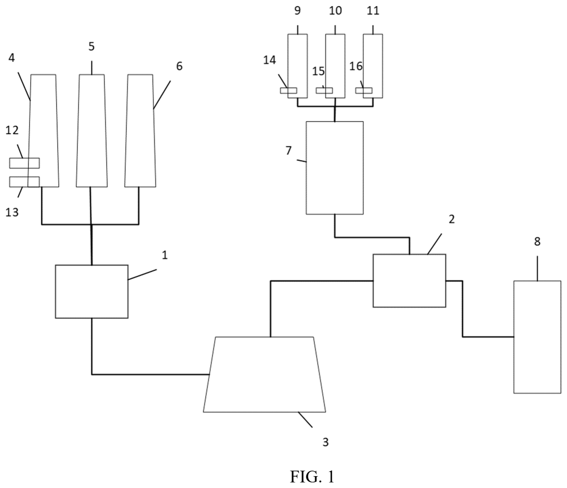

FIG. 1 is a schematic view of a dual pump harvesting apparatus provided in an embodiment of the present disclosure;
wherein, 1: first pump; 2: second pump; 3: centrifugal container; 4: initial sample container; 5: cleaning container; 6: resuspension container; 7: intermediate container; 8: waste liquid container; 9: product container; 10: product container; 11: product container; 12: bubble sensor; 13: weight sensor; 14: valve; 15: valve; 16: valve.

### DETAILED DESCRIPTION

To make the purpose, technical solution, and advantages of the present disclosure clearer, the technical solution of the present disclosure will be clearly and completely described below in conjunction with the drawings of the present disclosure. The described embodiments are part of the embodiments of the present disclosure, not all of the embodiments. Based on the embodiments of the present disclosure, all other embodiments obtained by ordinary technicians in this field without creative work are within the scope of protection of the present disclosure.

The present disclosure provides a dual pump harvesting process including a concentrating process and a formulating process.

In the concentrating process, two pumps are used, namely a first pump and a second pump. One end of the first pump is connected to an initial sample container and the other end of the first pump is connected to a centrifugal container. One end of the second pump is connected to the centrifugal container and the other end of the second pump is connected to an intermediate container and a waste liquid container. A flow rate difference between the two pumps during the concentrating process is set to be a flow rate threshold. A flow rate of the second pump is higher than that of the first pump to perform a continuous flow concentration.

Taking the apparatus shown in FIG. 1 as an example, the process of the dual pump harvesting method is as follows:
Step 1: consumable self-testing, including consumable entry testing, consumable sealing testing, waste liquid discharge testing, reference value testing, bubble sensor and/or pressure sensor performance testing, and continuous flow testing.
Step 2: parameter setting, including inputting a volume of an initial sample (0-99999 mL), determining a concentration way based on the inputted volume of the initial sample, and determining a condition for concentration based on a volume of a centrifugal container 3. In an embodiment, when the inputted volume of the initial sample is more than 12 times the volume of the centrifugal container 3, a circulating continuous flow concentration is used, otherwise a standard continuous flow concentration is used. For example, when the volume of the centrifugal container 3 is 1 L, the circulating continuous flow concentration is used when the volume of the initial sample is 12 L or more. The centrifugal container 3 can be selected from a variety type of devices that can perform centrifugation, such as centrifugal cups.

During the continuous flow concentration, the initial sample is pumped from an initial sample container 4 into the centrifuge container 3 through the first pump 1. During this process, the remaining volume of the initial sample in the initial sample container 4 is monitored. When the remaining volume falls below a volume threshold, a bubble sensor 12 is turned on to monitor whether the initial sample has been completely output from the initial sample container 4.

Further, the remaining volume of the initial sample in the initial sample container 4 can be monitored in other ways, such as by using a weight sensor 13. The bubble sensor 12 is turned on when the weight sensor 13 determines that the weight of the remaining initial sample in the initial sample container 4 falls below a weight threshold of the initial weight.

Further, in an embodiment, the volume threshold is preset to be within a range of 20% to 40% of the volume of the initial sample container 4. In another embodiment, the volume threshold can be 30% of the volume of the initial sample container 4.

### Step 3: Concentrating process

Based on the determination of the concentration way in the previous step, performing the standard continuous flow concentration, or the circulating continuous flow concentration. The steps for the standard continuous flow concentration are as follows.

Parameters of the standard continuous flow concentration: a centrifugal rotational speed of the centrifugal container 3 is 2200 rpm, the flow rate of the first pump 1 is 220 mL/min, and the flow rate of the second pump 2 is 400 mL/min. The Standard continuous flow concentration is carried out:

A preset volume of liquid is injected into the centrifugal container 3 for centrifugation. During the process, the first pump 1 pumps the liquid into the centrifugal container 3 at a flow rate of 220 mL/min, while the second pump 2 discharges the liquid from the centrifugal container 3 at a flow rate of 400 mL/min. The flow rate of the first pump 1 is less than that of the second pump 2, and the flow rate difference between the first pump and the second pump is equal to the flow rate threshold, thereby achieving the continuous flow concentration. Both the first pump and the second pump work uninterruptedly to achieve a concentration effect under the flow rate difference. In an embodiment, the flow rate threshold is 100-200 mL/min, which can be determined according to the volume of the initial sample during practical applications.

Furthermore, the liquid injection degree is monitored by the bubble sensor 12, and the standard continuous flow concentration is achieved once the liquid injection is complete.

### Parameters of the circulating continuous flow concentration

### 1. Determination of a number of concentration cycles

The number of concentration cycles is determined according to a volume of each concentration cycle. For example, when the volume of each concentration cycle is 5 L, the number of concentration cycles = the volume of the initial sample/5 L; when the volume of each concentration cycle is 4 L, the number of concentration cycles = the volume of the initial sample/4 L.

Each cycle of concentration is conducted in the same manner as the above-described standard continuous flow concentration, which is performed at a certain flow rate difference. The first pump 1 extracts the initial sample from the initial sample container 4 into the centrifugal container 3 for centrifugation, and then the second pump 2 pumps the supernatant into a waste liquid container 8. Until the remaining volume of a cell fluid in the centrifugal container 3 is lower than a concentration threshold, for example, 140-145 mL in the embodiment (the remaining volume is related to the volume of the centrifugal container 3 and can be preset, for example, when a large centrifugal container is used, the remaining volume can be set to be 140-145 mL, and when a small centrifugal container is used, the remaining volume can be set to be 70-75 mL), then the remaining cell fluid is extracted into an intermediate container 7. In an embodiment, the concentration threshold is 70-150 mL. The intermediate container 7 used in this embodiment of the disclosure can be an intermediate product bag.
2, Monitoring the progress by the bubble sensor 12 during the last cycle of concentration.
3, Ending

The cell fluid in the intermediate container 7 may be further concentrated by repeatedly using the above continuous flow concentration process.

### Step 4: Cleaning

After the concentrating process is completed, the cell fluid remaining in the centrifugal container 3 is cleaned. The cleaning process includes extracting a cleaning solution from a cleaning solution container 5 by the first pump 1, fusing the cleaning solution with other components in the cell fluid, and continuing the centrifugation, and pumping the supernatant to the waste fluid container 8 by the second pump 2. The cleaning degree can be determined according to the volume of the centrifugal container 3, for example, the remaining volume can be controlled to be 140-145 mL when a large centrifugal container is used, and the remaining volume can be controlled to be 70-5 mL when a small centrifugal container is used.

Furthermore, a number of cleaning times and the volume of a single cleaning can be preset, for example, the number of cleaning times can be set to be 0-10, and a single cleaning volume can be set to be 0-175 mL.

### Step 5: Cleaning with cryopreservation solution

After the previous cleaning step is completed, the cryopreservation solution can be selected for further cleaning, which includes the same steps as the previous cleaning step. The number of cleaning times can be preset to be 0-5, while the volume of a single cleaning can be preset be 0-400 mL (corresponding to a large centrifugal container) or 0-175 mL (corresponding to a small centrifugal container).

The cleaning degree can also be determined according to the volume of the centrifugal container 3, for example, when a large centrifugal container is used, the remaining volume can be controlled to be 90-100 mL, and when a small centrifugal container is used, the remaining volume can be controlled to be 40-45 mL.

### Step 6: Resuspending process

Firstly, the volume of concentrated cell fluid is obtained. At this time, the concentrated and cleaned cell fluid is in the centrifugal container 3, and a resuspension is pumped from a resuspension container 6 to the centrifugal container 3. The volume of the resuspension is preset, for example, to be 0-250 mL. After that, the resuspension and the cell fluid are mixed evenly. A mixing duration can be preset as well, for example, to be 300 s.

Secondly, after the mixing is completed, the sampling and counting can be carried out to calculate a cell density.

### Step 7: Dispensing of formulation

A dispensing process is determined by a total formulation volume, and a formulation threshold is set. A circulating formulating process is performed when the total formulation volume exceeds the formulation threshold, otherwise a single cycle formulating process is performed when the total formulation volume does not exceed the formulation threshold. For example, when the formulation threshold is set to be 250 mL, if the total formulation volume is less than or equal to 250 mL, the single cycle formulating process is performed; if the total formulation volume is greater than 250 mL, the circulating formulating process is performed.

### Single cycle formulating process

A volume of the resuspension for a second resuspending process is preset, which can be, for example, 0-250 mL, and the resuspension is drawn from the resuspension container 6 to the centrifugal container 3. The mixing duration can also be preset. After the mixing, the sampling and counting is carried out to calculate the cell density. Then the cell fluid is directly pumped into a product container (9, 10, or 11). The number and volume of the dispensing bag can be preset, for example, 1-9 dispensing bags are used, and the volume of each bag is 1-600 mL.

### Circulating formulating process

A total volume of a formula solution pumped into the intermediate container 7 is preset. The volume of the formula solution pumped into the intermediate container 7 for the first time is set at first. Then the formula solution is pumped into the centrifugal container 3 from the resuspension container 6 for performing the resuspending process, and the resuspended liquid is then directly pumped into the intermediate container 7. After that, the cups are cleaned, and the number of cleaning times is set to be 0-5, and the volume of the single cleaning is set to be 0-200 mL. The cleaning solution is directly pumped into the intermediate container 7. After the cleaning is completed, the sampling and counting is carried out again to determine the cell density, and the volume of the formula solution pumped into the intermediate container 7 for the second time is set, in which a compensation volume is added. The compensation volume is calculated by compensating 1 mL for each additional dispensing bags.

Further, after the above steps are completed, the formulation is dispensed from the intermediate container 7 into the product containers (9, 10, or 11) in a preset number and volume, for example, the number of the dispensing bags can be 1 to 9, and the volume of each bag can be 10 to 5000 mL.

Further, each product container corresponds to a valve, for example, the product container 9 corresponds to the valve 14, the product container 10 corresponds to the valve 15, and the product container 11 corresponds to the valve 16. In actual applications, when the product container is full, the corresponding valve is closed, and the product container is then replaced by a new one. After that, the valve is opened again to realize continuous dispensing.

Existing technology generally processes only the small-volume cell fluid, while the dual pump method provided by the present disclosure can process the high-flux cell fluid by utilizing two different concentration methods. This approach maintains a high flow rate, which allows for more efficient cell concentration, minimizes cell damage that can be caused by prolonged centrifugation and enhances both the yield and viability of the cells. The followings are the yield and viability of the cells processed using the dual pump method provided by the embodiments of the present disclosure. The parameters are optionally used in the actual applications. The results are shown in the following table:

**Table 1 Yield and viability of about 100 billion cells processed using the dual pump harvesting method**

| Batch | Sample | Cell Density | Sample Volume mL | Viability % | Cell number *10⁶ | Loss/Yield % | Cup | Cleaning solution |
|---|---|---|---|---|---|---|---|---|
| First time | T cell | 3.22E+06 | 3911.70 | 96.00% | 1.26E+ 11 | | Centrifugal container sample | NACL + Fetal bovine serum |
| | Waste liquid | 2.20E+04 | 4300.00 | | 9.46E+07 | 0.751% | | |
| | Harvest | 7.52E+07 | 157.60 | 95.26% | 1.19E+11 | 94.06% | | |
| Second time | T cell | 4.48E+06 | 1742.50 | 94.42% | 7.81E+10 | | Centrifugal container sample | NACL + Fetal bovine serum |
| | Waste liquid | 2.00E+04 | 2500.00 | | 5.00E+07 | 0.641% | | |
| | Harvest | 4.80E+07 | 150.00 | 93.35% | 7.20E+10 | 92.23% | | |

In the embodiment of the present disclosure, the dual pump harvesting method is used to carry out ten cycles of formulating process, the formulation volume is 1500 mL, and an inputted volume, a displayed volume, and an error of the valve 14, the valve 15, and the valve 16 are monitored. The results are shown in Table 1 to Table 5 as follows.

**Table 2 inputted volume, displayed volume, and error of the first to third cycles of formulating process**

| | First cycle | | | Second cycle | | | Third cycle | | |
|---|---|---|---|---|---|---|---|---|---|
| | Valve 14 | Valve 15 | Valve 16 | Valve 14 | Valve 15 | Valve 16 | Valve 14 | Valve 15 | Valve 16 |
| Inputted volume | 20 | 20 | 20 | 20 | 20 | 20 | 20 | 20 | 20 |
| Displayed volume | 21.1 | 22.3 | 21.8 | 21.2 | 22 | 22 | 21.8 | 22.3 | 21.8 |
| Actual volume | 21.5 | 21.8 | 22.1 | 21.4 | 21.7 | 22.4 | 21.9 | 22.1 | 22.1 |
| Error | 0.4 | -0.5 | 0.3 | 0.2 | -0.3 | 0.4 | 0.1 | -0.2 | 0.3 |

**Table 3 inputted volume, displayed volume, and error of the fourth to fifth cycles**

| | Fourth cycle | | | Fifth cycle | | |
|---|---|---|---|---|---|---|
| | Valve 14 | Valve 15 | Valve 16 | Valve 14 | Valve 15 | Valve 16 |
| Inputted volume | 20 | 20 | 20 | 20 | 20 | 20 |
| Displayed volume | 21.5 | 22.2 | 22 | 21.6 | 21.9 | 22.2 |
| Actual volume | 21.8 | 22.2 | 21.9 | 22 | 22.2 | 21.9 |
| Error | 0.3 | 0 | -0.1 | 0.4 | 0.3 | -0.3 |

**Table 4 inputted volume, displayed volume, and error of the sixth to eighth cycles of formulating process**

| | Sixth cycle | | | Seventh cycle | | | Eighth cycle | | |
|---|---|---|---|---|---|---|---|---|---|
| | Valve 14 | Valve 15 | Valve 16 | Valve 14 | Valve 15 | Valve 16 | Valve 14 | Valve 15 | Valve 16 |
| Inputted volume | 20 | 20 | 20 | 20 | 20 | 20 | 20 | 20 | 20 |
| Displayed volume | 21.3 | 22.4 | 22.4 | 21.7 | 22.3 | 21.9 | 21.6 | 22.3 | 21.5 |
| Actual volume | 22.1 | 22.6 | 22 | 21.8 | 22.1 | 21.8 | 22 | 22.5 | 22.4 |
| Error | 0.8 | 0.2 | -0.4 | 0.1 | -0.2 | -0.1 | 0.4 | 0.2 | 0.9 |

**Table 5 inputted volume, displayed volume, and error of the ninth and tenth cycles of formulating process**

| | Ninth cycle | | | Tenth cycle | | |
|---|---|---|---|---|---|---|
| | Valve 14 | Valve 15 | Valve 16 | Valve 14 | Valve 15 | Valve 16 |
| Inputted volume | 20 | 20 | 20 | 20 | 20 | 20 |
| Displayed volume | 21.3 | 22.5 | 22.1 | 21.7 | 22 | 21.8 |
| Actual volume | 22.1 | 22.3 | 22 | 22.1 | 21.7 | 21.9 |
| Error | 0.8 | -0.2 | -0.1 | 0.4 | -0.3 | 0.1 |

The formulations obtained in embodiments of the present disclosure are further compared with existing formulations obtained without using the dual pump harvesting method. The results of the effect of the formulations using the existing single pump harvesting method are shown in Table 6, which shows that the errors of the formulations obtained by using the single pump harvesting method are significantly higher than the errors of the formulations obtained using the dual pump harvesting method provided by embodiments of the present disclosure.

**Table 6 Single pump harvesting method**

| Formulation | Target value | Displayed volume (mL) | | | Actual volume (mL) | | | Error (mL) | | |
|---|---|---|---|---|---|---|---|---|---|---|
| | | First bag | Second bag | Third bag | First bag | Second bag | Third bag | First bag | Second bag | Third bag |
| First time | 20 | 21.2 | 22.8 | 23.1 | 20.3 | 21 | 23.6 | 0.3 | 1 | 3.6 |
| Second time | | 21.8 | 22.3 | 21.9 | 20.2 | 21.1 | 22.4 | 0.2 | 1.1 | 2.4 |
| Third time | | 21.8 | 22.8 | 21.6 | 20.1 | 22.5 | 23.3 | 0.1 | 2.5 | 3.3 |

Finally, it should be noted that the above embodiments are only used to illustrate the technical solutions of the present disclosure, rather than to limit it. Although the present disclosure has been described in detail with reference to the embodiments, those skilled in the art should understand that they can still modify the technical solutions described in the embodiments, or make equivalent replacements for some of the technical features therein. However, these modifications or replacements do not deviate the essence of the corresponding technical solutions from the spirit and scope of the technical solutions of the embodiments of the present disclosure.

### Industrial utility

The present disclosure provides the dual pump harvesting method and apparatus. The dual pump harvesting method includes the concentrating process and the formulating process. The concentrating process includes using two pumps to perform the concentrating process. One end of the first pump is connected to the initial sample container and the other end thereof is connected to the centrifugal container; one end of the second pump is connected to the centrifugal container and the other end thereof is connected to the intermediate container and the waste liquid container. The flow rate difference between the second pump and the first pump during the concentrating process is set to be the flow rate threshold, and the flow rate of the second pump is higher, thereby performing the continuous flow concentration. The present disclosure realizes the circulating continuous flow concentration through a specific dual pump process. Combined with the subsequent circulating formulation, a cell formulation method is obtained, which does not require manual operation to achieve the function of multiple formulation bags for formulation, and improves the formulation efficiency and precision. It is of great significance in the field of cellular drug production and has good economic value and application prospects.

## Claims

1. A dual pump harvesting method, comprising a concentrating process and a formulating process, wherein the concentrating process comprises:
using two pumps to perform the concentrating process, wherein one end of a first pump of the two pumps is connected to an initial sample container and an other end of the first pump is connected to a centrifugal container; one end of a second pump of the two pumps is connected to the centrifugal container and an other end of the second pump is connected to an intermediate container and a waste liquid container; and
setting a flow rate difference between the first pump and the second pump to be a flow rate threshold, and setting a flow rate of the second pump to be higher than that of the first pump to perform a continuous flow concentration.

2. The dual pump harvesting method according to claim 1, wherein the flow rate of the first pump is 100-200 mL/min, and/or the flow rate of the second pump is 200-300 mL/min.

3. The dual pump harvesting method according to claim 1 or 2, wherein the first pump extracts an initial sample from the initial sample container into the centrifugal container for centrifugation, and the second pump pumps a supernatant in the centrifugal container into the waste liquid container, thereby forming the continuous flow concentration until a remaining volume of a cell fluid in the centrifugal container is lower than a concentration threshold.

4. The dual pump harvesting method according to claim 3, wherein during a circulating continuous flow concentration, a concentration volume is preset, and a number of concentration cycles is determined based on the concentration volume.

5. The dual pump harvesting method according to any one from claim 1 to 4, wherein the concentrating process further comprises determining a concentration way based on a volume of the initial sample; when the volume of the initial sample is greater than a preset multiple of a volume of the initial sample container, a circulating continuous flow concentration is used, otherwise a standard continuous flow concentration is used.

6. The dual pump harvesting method according to claim 1, wherein during the concentrating process, the initial sample in the initial sample container is pumped to the centrifugal container, and when an amount of the initial sample remaining in the initial sample container is reduced to a preset percentage, a bubble sensor is turned on.

7. The dual pump harvesting method according to claim 1, wherein the formulating process comprises adding a first preset volume of a resuspension into the centrifugal container to resuspend cells and then determining the formulating process according to a preset total formulation volume;
when the preset total formulation volume is less than 250 mL, a single cycle formulating process is used;
when the preset total formulation volume is greater than 250 mL, a circulating formulating process is used.

8. The dual pump harvesting method according to claim 7, wherein the circulating formulating process comprises:
adding a first preset volume of formula solution to the intermediate container; transferring resuspended cells in the centrifugal container to the intermediate container; cleaning the centrifugal container and transferring a used cleaning solution to the intermediate container; adding a second preset volume of the formula solution to the intermediate container such that a total volume of a cell fluid in the intermediate container is equal to a sum of the preset total formulation volume and a compensation volume;
the cell fluid in the intermediate container is dispensed according to a preset dispensing quantity and a preset dispensing volume.

9. A dual pump harvesting apparatus, comprising a first pump and a second pump, wherein:
one end of the first pump is connected to an initial sample container and an other end of the first pump is connected to a centrifugal container; one end of the second pump is connected to the centrifugal container and an other end of the second pump is connected to an intermediate container and a waste liquid container;
a flow rate difference between the second pump and the first pump during concentration is set to be a flow rate threshold, and a flow rate of the second pump is higher than that of the first pump to perform a continuous flow concentration.

10. A cell formulating system, wherein the cell formulating system comprises the dual pump harvesting apparatus according to claim 9.
